(19) Europäisches Patentamt
European Patent Office
Office européen des brevets

(11) **EP 1 532 925 A1**

(12) **EUROPEAN PATENT APPLICATION**

(43) Date of publication:
**25.05.2005 Bulletin 2005/21**

(51) Int Cl.⁷: **A61B 5/15**

(21) Application number: **04257169.5**

(22) Date of filing: **19.11.2004**

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: **21.11.2003 US 718818**

(71) Applicant: **LIFESCAN, INC.**
**Milpitas, California 95035 (US)**

(72) Inventors:
• **Stiene, Matthias**
  **82205 Gilching (DE)**
• **Hilgers, Michael Edward**
  **LakeELmo, MN 55042 (US)**
• **Richter, Tanja**
  **Inverness-shire IV2 3LJ (GB)**

(74) Representative: **Mercer, Christopher Paul et al**
**Carpmaels & Ransford,**
**43-45 Bloomsbury Square**
**London WC1A 2RA (GB)**

(54) **Device and method for extracting body fluid**

(57)    A device for extracting bodily fluid such as an ISF sample includes a penetration member (402) with a channel, e.g., a hollow needle and a fluid flow regulator (404) for example, a narrow-bore cylinder disposed within the channel. The penetration member is configured for penetrating a target site such as a dermal tissue target site and subsequently residing within the target site and extracting a bodily fluid sample therefrom. The fluid flow regulator is adapted to control, e.g., reduce or minimize variation in bodily fluid flow rate through the penetration member. In addition, the presence of the fluid flow regulator in the channel of the penetration member serves to reduce sensor lag by reducing the dead volume of the penetration member. A method for extracting bodily fluid from a target site includes providing the aforementioned device. Next, the target site is penetrated with the penetration member of the device. Subsequently, bodily fluid is extracted from the target site via the penetration member and the fluid flow regulator of the device.

FIG. 5

**Description**

BACKGROUND OF INVENTION

1. Field of the Invention

The present invention relates, in general, to devices and methods for extracting bodily fluid and, in particular, to devices and methods for extracting bodily fluid in a continuous or semi-continuous manner.

2. Description of the Related Art

In recent years, efforts in medical devices for monitoring the concentration of analytes (e.g., glucose) in bodily fluids (e.g., blood and interstitial fluid) have been directed toward developing devices and methods that allow continuous or semi-continuous monitoring.

In the context of blood glucose monitoring, continuous or semi-continuous monitoring devices and methods are advantageous in that they provide enhanced insight into blood glucose concentration trends, the effect of food and medication on blood glucose concentration and a user's overall glycemic control. In practice, however, continuous and semi-continuous monitoring devices can have drawbacks. For example, during extraction of an interstitial fluid (ISF) sample from a target site (e.g., a user's dermal tissue target site) via a sampling module of a medical device, ISF flow rate can vary and/or decay over time.

Furthermore, continuous and semi-continuous monitoring devices can suffer from a deleterious effect known as "sensor lag." Such a sensor lag effect occurs when a significant difference exists between an analyte concentration at a sensor of the continuous monitoring device and the real-time analyte concentration at the target site.

Still needed in the field, therefore, is a device and associated method for extracting bodily fluid (such as ISF) that facilitate continuous or semi-continuous monitoring of the extracted bodily fluid while minimizing bodily fluid flow rate variation and decay and/or reducing sensor lag effect.

SUMMARY OF INVENTION

Devices for extracting bodily fluid according to embodiments of the present invention facilitate continuous or semi-continuous monitoring of the extracted bodily fluid while minimizing bodily fluid flow rate variation and decay and/ or reducing sensor lag.

Devices for extracting bodily fluid (such as an ISF sample) according to embodiments of the present invention include a penetration member with a channel (e.g., a hollow needle) and a flow regulator (such as a narrow-bore cylinder) disposed within the channel. The penetration member is configured for penetrating a target site (e.g., a dermal tissue target site) and subsequently residing within the target site and extracting a bodily fluid sample therefrom. The fluid flow regulator is adapted to control (e.g., reduce or minimize) variation in bodily fluid flow rate through the penetration member. In addition, the presence of the fluid flow regulator in the channel of the penetration member serves to reduce sensor lag by reducing a dead volume of the penetration member.

A method for extracting bodily fluid from a target site according to an embodiment of the present invention includes providing a device for extracting bodily fluid (in accordance with the present invention as described herein). The target site is then penetrated with a penetration member of the device, followed by extraction of bodily fluid from the target site via the penetration member and a fluid flow regulator of the device.

BRIEF DESCRIPTION OF DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a simplified block diagram depicting a system for extracting a bodily fluid sample and monitoring an analyte concentration therein as may be used in conjunction with embodiments of the present invention;
FIG. 2 is a simplified schematic diagram of an ISF sampling module being applied to a user's dermal tissue (skin) target site as may be used in conjunction with embodiments of the present invention, with the dashed arrow indicating a mechanical interaction and the solid arrows indicating either ISF flow or, when associated with element 28, the application of pressure;
FIG. 3 is a simplified cross-sectional side view of an extraction device in combination with a device for extracting bodily fluid according to an exemplary embodiment of the present invention;

FIG. 4 is a simplified perspective view of a device for extracting bodily fluid according to an exemplary embodiment of the present invention;

FIG. 5 is a simplified cross-sectional view of the device of FIG. 4 taken along line 5A-5A of FIG. 4;

FIG. 6 is a simplified cross-sectional view of a device according to another exemplary embodiment of the present invention;

FIG. 7 is a simplified cross-sectional view of a device according to yet another exemplary embodiment of the present invention;

FIG. 8 is a graph showing fluid flow rate as a function of time using a conventional penetration member and an externally disposed flow regulator in comparison to a conventional penetration member;

FIG. 9 is a graph showing volume of fluid collected as a function of time using a conventional penetration member and an externally disposed flow regulator in comparison to a conventional penetration member; and

FIG. 10 is a flow diagram illustrating a sequence of steps in a process according to an exemplary embodiment of the present invention.

## DETAILED DESCRIPTION OF THE INVENTION

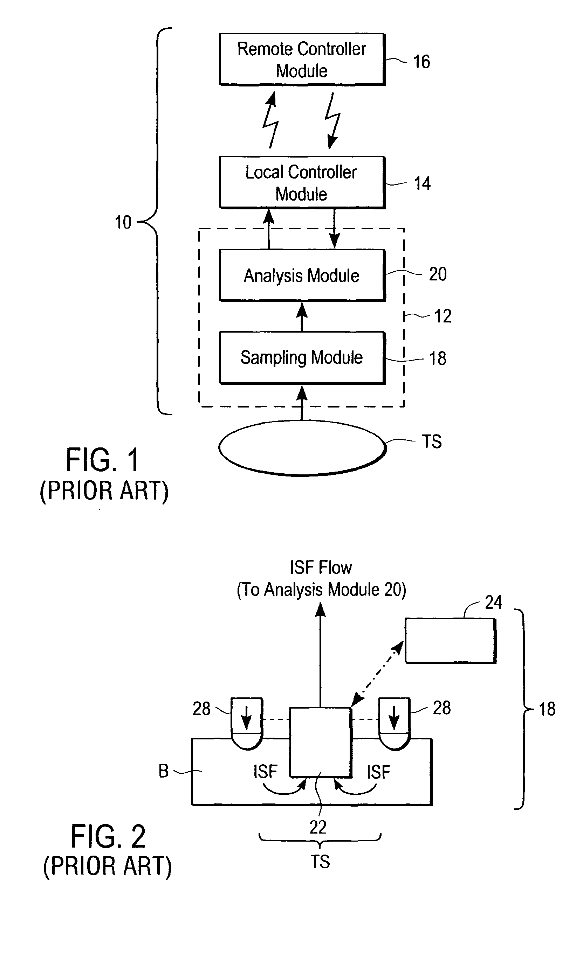

Devices for extracting bodily fluid according to embodiments of the present invention can be readily employed in conjunction with systems for extracting a bodily fluid sample (e.g., an ISF sample) and monitoring a concentration of an analyte (e.g., glucose) therein, ISF extraction devices and other suitable medical devices known to those of skill in the art. For example, FIG. 1 depicts a system 10 for extracting an ISF sample that includes a disposable cartridge 12 (encompassed within the dashed box), a local controller module 14 and a remote controller module 16.

In system 10, disposable cartridge 12 includes a sampling module 18 for extracting the bodily fluid sample (e.g., an ISF sample) from a target site TS (for example, a user's dermal tissue target site) and an analysis module 20 for measuring a concentration of an analyte (i.e., glucose) in the bodily fluid. Further details regarding system 10 are described in U.S. Patent Application No. 10/653,023. In addition, examples of sampling and analysis modules are described in International Application PCT/GB01/05634 (published as WO 02/49507 on June 27, 2002).

As depicted in FIG. 2, sampling module 18 of system 10 can include a penetration member 22 for penetrating the target site (TS) and extracting an ISF sample, a launching mechanism 24 and at least one pressure ring 28. U.S. Patent Application No. 10/653,023 describes the manner in which sampling module 18 is adapted to provide a continuous or semi-continuous flow of ISF to analysis module 20 for the monitoring (e.g., concentration measurement) of an analyte (such as glucose) in the ISF sample.

During use of system 10, penetration member 22 is inserted into the target site (i.e., penetrates the target site) by operation of launching mechanism 24. For the extraction of an ISF sample from a user's skin layer, penetration member 22 can be inserted to a maximum insertion depth in the range of, for example, 1.5 mm to 3 mm. Penetration member 22 of such a conventional system typically consists of a 25 gauge, thin-wall stainless steel needle with a bent tip, wherein a fulcrum for the tip bend is disposed between the needle's tip and the needle's heel. Further examples of such conventional penetration members are described in U.S. Patent Application Publication No. 2003/0060784A1.

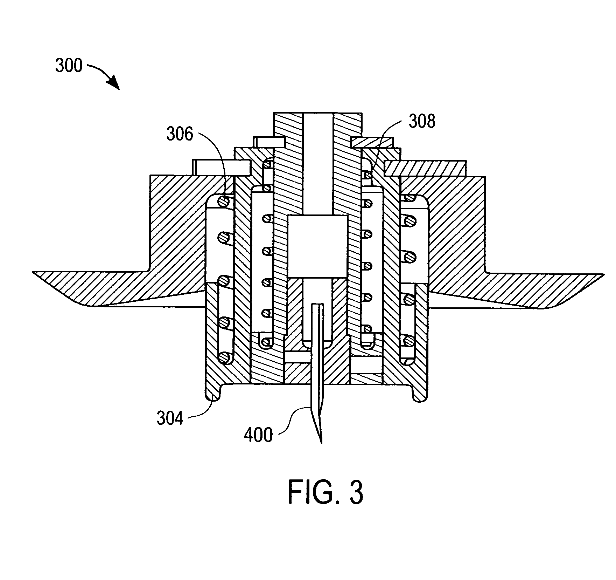

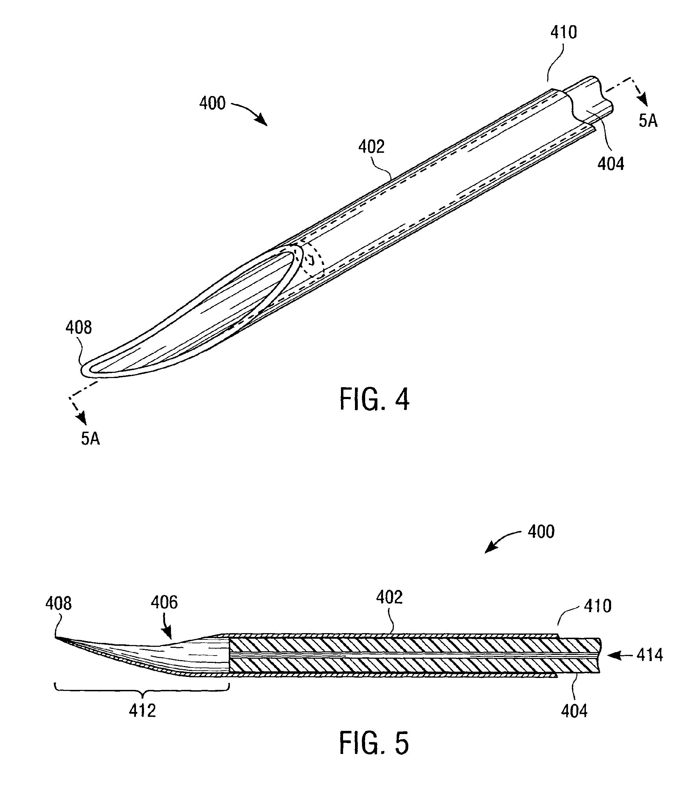

FIG. 3 is a cross-sectional side view of an interstitial fluid (ISF) extraction device 300 that can be used in conjunction with embodiments of the present invention. ISF extraction device 300 includes an oscillatable pressure ring 304, a first biasing member 306 (i.e., a first spring) and a second biasing member 308 (i.e., a second spring). FIG. 3 depicts extraction device 300 in use with a device 400 (also illustrated in FIGs. 4 and 5) for extracting bodily fluid according to an exemplary embodiment of the present invention.

Referring to FIGs. 3, 4 and 5, device 400 is adapted for extracting bodily fluid (e.g., ISF) and includes a penetration member 402 and a fluid flow regulator 404. Penetration member 402 has a channel 406 therethrough and is configured for penetrating a target site (such as a user's dermal tissue target site). Subsequent to such a penetration, penetration member 402 resides within the target site and extracts a bodily fluid sample from the target site via channel 406.

Penetration member 402 is essentially a hollow tube (e.g., a hollow needle) with a distal end 408 and a proximal end 410. Distal end 408 is configured as a sharp point for penetrating a target site. One skilled in the art will recognize that proximal end 410 can, for example, be connected to analysis module 20 of system 10 (see FIG. 1) and be in fluid communication therewith.

As noted above, penetration member 402 is configured to remain in (reside in) the target site during the extraction of a bodily fluid sample therefrom. Penetration member 402 can, for example, remain in the target site for more than one hour, thus allowing a continuous or semi-continuous extraction of a bodily fluid sample, such as ISF. Once apprised of the present disclosure, one skilled in the art will recognize that the penetration member can reside in the target site for an extended period of time of 8 hours or more.

The configuration of penetration member 402 is adapted to optimize ISF collection and includes a bent tip 412. Penetration member 402 can be formed of, for example, stainless steel or a biocompatible high-strength polymer (e.g., a biocompatible high-strength liquid crystal polymer). Typical, but non-limiting, dimensions of a penetration member are an outer diameter (OD) in the range of approximately 300 μm to approximately 700 μm, an inner diameter (ID) in the range of from approximately 100 μm to approximately 500 μm and a length in the range of approximately 3 mm to approximately 30 mm.

Fluid flow regulator 404 is disposed within the channel 406 of penetration member 402 and is adapted to minimize variation in bodily fluid flow through channel 406. In the embodiment of FIGs. 3, 4 and 5, fluid flow regulator 404 is a cylindrical in form and includes a narrow-bore channel 414. Narrow-bore channel 414 serves to provide an increased hydraulic resistance to the flow of bodily fluid through device 400.

The cross-sectional dimensions of narrow-bore channel 414 are predetermined such that a consistent flow of bodily fluid through device 400 is obtained. In this regard, typical ISF flow rates in devices according to present invention are in the range of, for example, 20 nanoliters to 200 nanoliters per minute.

In addition, since fluid flow regulator 404 is disposed within channel 406, the 'dead volume' of penetration member 402 is beneficially reduced. The dead volume of a penetration member is the volume of ISF or other bodily fluid that is contained within the penetration member. Such a dead volume results in a lag between the point in time when a bodily fluid sample enters the penetration member and the point in time when the bodily fluid sample exits the penetration member for further manipulation (e.g., measurement of analyte concentration). A result of such a dead volume is sensor lag. For example, if the dead volume is 1000 nanoliters and the bodily fluid flow rate is 100 nanoliters per minute, then the sensor lag is 10 minutes.

From the above discussion, it is evident that a reduction of dead volume will result in a beneficial reduction in sensor lag. Therefore, the disposition of fluid flow regulator 404 within channel 406 serves to beneficially reduce both dead volume and sensor lag.

It should be noted that disposing a fluid flow regulator within the channel of a penetration member, rather then simply providing a penetration member with a relatively narrow channel, provides for the penetration member to have an opening at distal end 408 with a diameter that is relatively large in comparison to the diameter of narrow-bore channel 414. Such a relatively large opening can be beneficial in facilitating extraction of a bodily fluid sample through the device.

Fluid flow regulator 404 also serves to limit the flow of bodily fluid through penetration member 402 by constricting the path (i.e, the penetration member's channel) through which the bodily fluid can flow. In the absence of a fluid flow regulator, bodily fluid flow through the channel of a penetration member would initially proceed at a relatively high flow rate and then decline as bodily fluid in the target site (e.g., a user's dermal tissue target site) is depleted. Such a relatively high initial flow rate can be greater than is needed to supply an associated analysis system (e.g., analysis module 20 of FIG. 1) with adequate bodily fluid for a continuous measurement. After a period of time (e.g., approximately one to three hours), the bodily fluid flow rate can decrease below the minimum flow rate required for operation of the associated analysis system.

However, it has been determined that the presence of a fluid flow regulator within the channel of the penetration members results in a more steady bodily fluid flow rate and a bodily fluid flow rate that remains at a level consistent with proper and accurate operation of an associated analysis system for an extended period of time. Since electrochemical measurement systems are more accurate and precise if the flow rate past a sensor of the electrochemical measurement system is constant, the consistent flow rates obtained with devices according to the present invention are expected to result in increased accuracy and precision of analyte measurements.

The dimensions of narrow-bore channel 414 can be predetermined to meet the minimum flow rate for an associated analytical system, with which device 400 is to be employed. Typical diameters for narrow-bore channel 414, however, are in the range of from about 5 μm to 150 μm. The cross-sectional shape of narrow-bore channel 414 can be, but is not limited to circular, oval, elliptical, square or rectangular shapes.

The length of fluid flow regulator 404 can also be predetermined to meet the minimum flow rate for an associated analytical system. Typical lengths of fluid flow regulator 404, however, are in the range of from about 5 millimeters to 100 millimeters.

Once apprised of the present disclosure, one skilled in the art will recognize that the ability of a given fluid flow regulator to control fluid flow through a penetration member is a function of the fluid flow regulator's flow resistance R. Major factors affecting flow resistance R are the radius (r) and length (L) of the narrow-bore channel with the resistance R for a straight narrow-bore channel being defined as follows: $R = 8L \pi r^4$.

The reduction in dead volume achieved by disposing fluid flow regulator 404 in channel 406 is a function of the theoretical dead volume of penetration member 402 in the absence of fluid flow regulator 404, the diameter of channel 406 and the diameter of narrow-bore channel 414. The dead volume of device 400 can be represented by the following equation (that disregards any volumes present beyond the fluid flow regulator at the distal and proximal ends of the device):

$$Volume_{device\_400} = Volume_{theoretical} *(Diameter_{regulator}/Diameter)^2$$

where:

$$Volume_{device\_400} = \text{dead volume of device 400;}$$

$$Volume_{theoretical} = \text{volume of penetration member 402 in the absence of fluid flow regulator 404;}$$

$$Diameter_{regulator} = \text{internal diameter of narrow-bore channel 414}$$

$$Diameter = \text{internal diameter of channel 406.}$$

Per the immediately above equation, a four-fold reduction in internal diameter results in a sixteen-fold reduction in dead volume for device 400. Thus, a significant reduction in dead volume can be achieved with the use of fluid flow regulator 404.

Fluid flow regulator 404 can be formed, for example, of stainless steel or a suitable biocompatible material including, but not limited to, biocompatible polymers such as polytetrafluoroethylene (PTFE), fluorinated ethylene propylene (FEP), polyether ether ketone (PEEK), polyurethane and silicone. Fluid flow regulator 404 can, if desired, be formed of an anti-thrombogenic material or non-thrombogenic material, thus potentially simplifying manufacturing by eliminating any need to provide penetration member 402 with anti or non-thrombogenic properties. However, fluid flow regulator 404 and penetration member 402 can, if desired, be formed as an integral unit (e.g., an integral molded unit).

The surface(s) of the fluid flow regulator that come into contact with bodily fluid during use can be optionally coated with a non-thrombogenic or anti-thrombogenic material to prevent clogging of the fluid flow regulator during use of the device. Examples of suitable non-thrombogenic and anti-thrombogenic materials include, olyurethane, polytetrafluoroethylene (PTFE), polyvinyl pyrrolidone (PVP), heparin benzalkonium chloride, hirudin, salicylic acid (aspirin) or EDTA. Once apprised of the present disclosure, those skilled in the art will also recognize that the polymeric material of which the fluid flow regulator can be formed may contain one or more non-thrombogenic or anti-thrombogenic materials embedded therein or bound thereto.

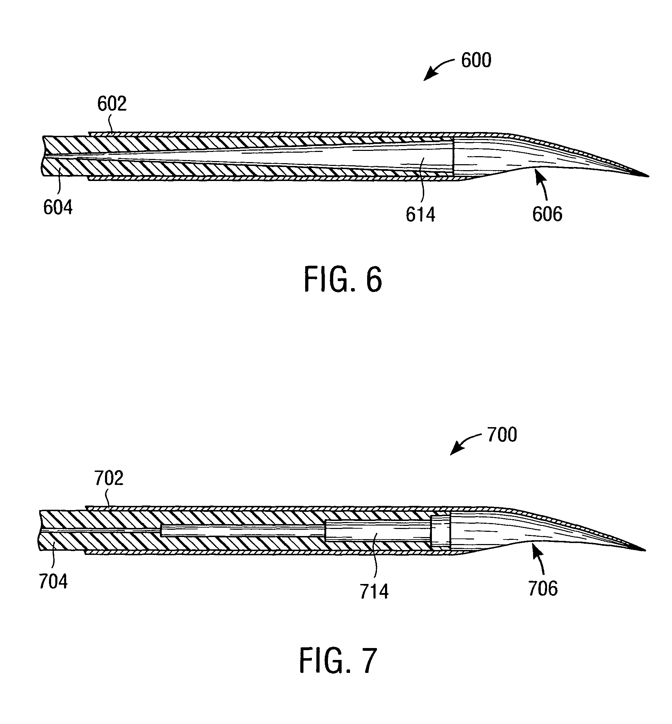

Referring to FIG. 6, device 600 adapted for extracting bodily fluid according to another exemplary embodiment of the present invention includes a penetration member 602 and a fluid flow regulator 604. Penetration member 602 has a channel 606 therethrough and is configured for penetrating a target site (such as a user's dermal tissue target site). Subsequent to such a penetration, penetration member 602 resides within the target site and extracts a bodily fluid sample from the target site via channel 606.

Fluid flow regulator 604 is disposed within the channel 606 of penetration member 602 and is adapted to minimize variation in bodily fluid flow through channel 606. In the embodiment of FIG. 6, fluid flow regulator 604 is a cylindrical in form and includes a narrow-bore channel 614 with a gradually decreasing diameter.

Referring to FIG. 7, device 700 adapted for extracting bodily fluid according to yet another exemplary embodiment of the present invention includes a penetration member 702 and a fluid flow regulator 704. Penetration member 702 has a channel 706 therethrough and is configured for penetrating a target site (such as a user's dermal tissue target site). Subsequent to such a penetration, penetration member 702 resides within the target site and extracts a bodily fluid sample from the target site via channel 706.

Fluid flow regulator 704 is disposed within the channel 706 of penetration member 702 and is adapted to minimize variation in bodily fluid flow through channel 706. In the embodiment of FIG. 7, fluid flow regulator 704 is a cylindrical in form and includes a narrow-bore channel 714 with a diameter that decreases in a step-wise manner.

Once apprised of the present invention, one skilled in the art will recognize that devices according to embodiments of the present invention can be used in conjunction with a variety of systems (e.g., the system of FIGs. 1 and 2) and ISF extraction devices (for example, the ISF extraction device of FIG. 3). In this regard, devices according to the present invention can be beneficially employed in conjunction with extraction devices that include oscillatable pressure rings. During such use, devices according to embodiments of the present invention can be employed to smooth out the fluctuation in bodily fluid flow rate caused by the oscillation (i.e., deployment and retraction) of the pressure rings, thereby maintaining the bodily fluid flow rate at a level required by an associated analysis system module.

**Example 1: Fluid Flow Rate versus Time**

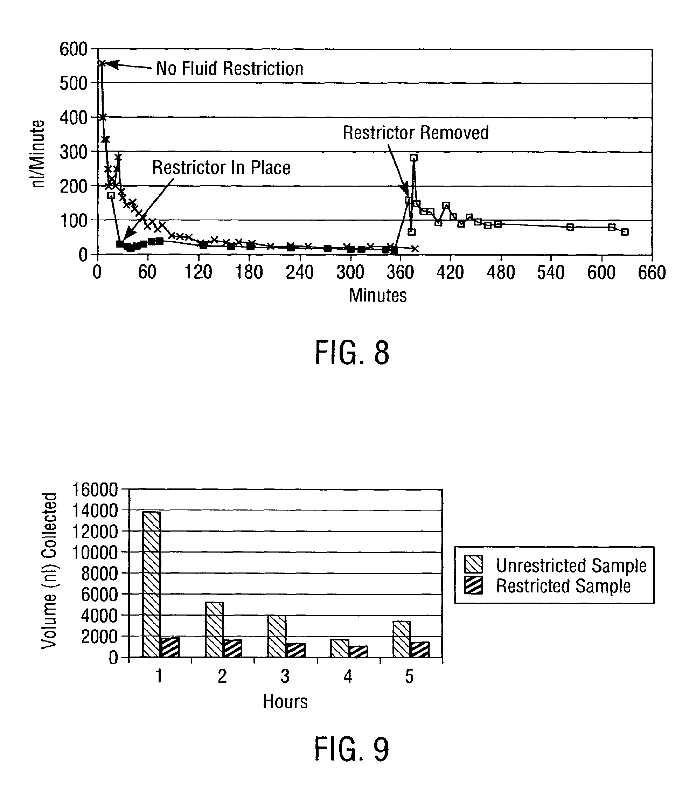

FIG. 8 illustrates ISF flow rate through a 25-guage (nominal channel inner diameter of 310 µm , 32 mm length) penetration member in the absence ("x" and open square symbols) and presence (closed square symbols) of an externally disposed fluid flow regulator with a narrow-bore channel (namely a 12 µm height and a 15 µm width narrow-bore channel, 8 mm length) versus time. Flow rate was determined by measuring the distance that a leading edge of ISF traveled over time. The dimensions of either the 25-guage penetration member or narrow-bore channel were then used to calculate the ISF flow rate. Although the fluid flow regulator was externally disposed, it is postulated, without being bound, that the results of this study are indicative of the behavior for a device with a fluid flow regulator disposed within the penetration member.

The data represented by "x" symbols were generated for the penetration member in the absence of a fluid flow regulator. Up until approximately 90 minutes, the ISF flow rate was much greater than needed to supply a typical analysis system with adequate ISF for a continuous or semi-continuous measurement of an analyte. After approximately 90 minutes, the ISF flow rate decreased to a rate of approximately 20 nanoliters per minute.

The data represented by open squares were also generated for a penetration member in the absence of a fluid flow regulator. The initial flow rate in the absence of a fluid flow regulator was approximately 170 nanoliters per minute. The fluid flow regulator was then inserted into the channel of the penetration member. With the fluid flow regulator in place, the ISF flow rate slowed and remained relatively constant at approximately 20 nanoliters per minute for an extended duration (i.e., the duration from about 20 minutes until about 360 minutes in FIG. 8). These data are represented by the closed squares in FIG. 8. The fluid flow regulator was then removed from the penetration member and the ISF flow rate increased to approximately the same level as before the fluid flow regulator was in place.

**Example 2: Volume of ISF Collected versus Time Period**

FIG. 9 is a bar chart of the volume of ISF collected over one hour time periods as calculated based on the data of FIG. 8. Approximately half of the total ISF volume collected in the absence of a fluid flow regulator was collected in the first 60 minutes. The fluid collection in the presence of a fluid flow regulator was evenly distributed throughout the time that the fluid flow regulator was in place.

In FIG. 9, the total volume of ISF collected in the absence of a fluid flow regulator was about 28000 nanoliters. If an associated analysis system requires 50 nanoliters per minute of ISF, the analysis system would only function for 90 minutes before the ISF flow rate would fall below the minimum requirement (see FIG. 9). If an appropriately sized fluid flow regulator were present, ISF could be collected at 50 nanoliters per minute and the analysis system would function for 560 minutes. The data in FIG. 9 show that the total volume collected in the presence of the fluid flow regulator was about 3500 nanoliters, which equates to 700 minutes at a flow rate of 50 nanoliters per minute.

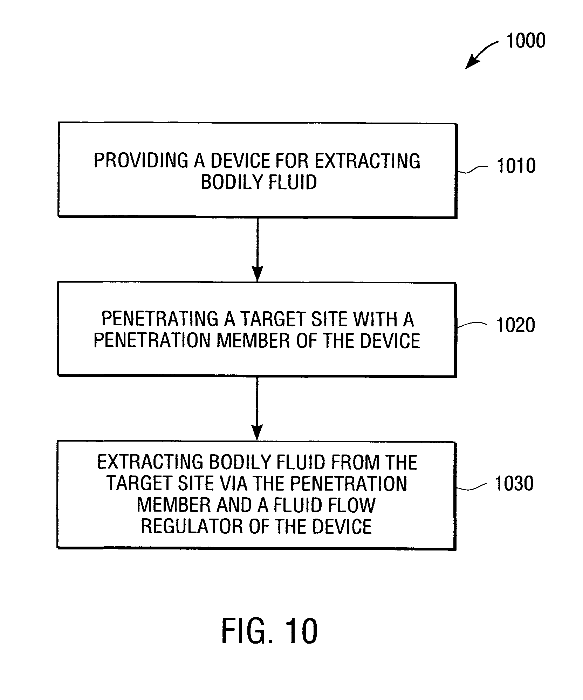

Referring to FIG. 10, a method 1000 for extracting a bodily fluid includes providing a device for extracting bodily fluid according to the present invention as described above, as set forth in step 1010. Such a device includes a penetration member with a channel (e.g., a hollow needle) and a flow regulator (e.g., a narrow-bore cylinder) disposed within the channel. The penetration member is configured for penetrating a target site (such as a dermal tissue target site) and subsequently residing within the target site and extracting a bodily fluid sample therefrom. The fluid flow regulator is adapted to control (for example, reduce or minimize variation in) bodily fluid flow rate through the penetration member. In addition, the presence of the fluid flow regulator in the channel of the penetration member serves to reduce sensor lag by reducing a dead volume of the penetration member.

Next, at step 1020, the target site is penetrated by the penetration member. Subsequently, a bodily fluid sample is extracted from the target site via the penetration member and fluid flow regulator, as set forth in step 1030.

It should be understood that various alternatives to the embodiments of the invention described herein may be employed in practicing the invention. It is intended that the following claims define the scope of the invention and that methods and structures within the scope of these claims and their equivalents be covered thereby.

**Claims**

**1.** A device for extracting bodily fluid, the device comprising:

a penetration member, the penetration member having a channel and being configured for penetrating a target site and subsequently residing within the target site and extracting a bodily fluid sample therefrom; and a fluid flow regulator disposed within the channel of the penetration member, the fluid flow regulator adapted to reduce bodily fluid flow rate through the penetration member.

2. The device of claim 1, wherein the fluid flow regulator is further adapted to minimize bodily fluid flow rate variation through the penetration member.

3. The device of claim 1 or claim 2, wherein the fluid flow regulator is further adapted to optimize a dead volume of the device.

4. The device of any one of claims 1 to 4, wherein the penetration member is configured for penetrating a dermal tissue target site and extracting an interstitial fluid sample therefrom.

5. The device of any one of claims 1 to 4, wherein the channel has an inner diameter in the range 100 μm to 500 μm.

6. The device of any one of claims 1 to 5, wherein the fluid flow regulator includes a narrow-bore channel and wherein a diameter of the narrow-bore channel is less than a diameter of the channel.

7. The device of claim 6, wherein the narrow-bore channel has a diameter in the range of 5 μm to 150 μm.

8. The device of claim 6, wherein the narrow-bore channel has a gradually decreasing diameter.

9. The device of claim 6, wherein the narrow-bore channel has a diameter that decreases in a stepped manner.

10. The device of claim 6, wherein the diameter of the channel of the penetration member is approximately 300 μm and the narrow-bore channel has a width of 12 μm and a height of 15 μm.

11. The device of any one of claims 1 to 10 further including a non-thrombogenic coatings or anti-thrombogenic coating on at least one surface of the fluid flow regulator.

12. The device of any one of claims 1 or 11, wherein the penetration member and the fluid flow regulator are formed as an integral unit.

13. The device of any one of claims 1 to 12, wherein the penetration member is formed of stainless steel and the fluid flow regulator is formed of a polymer.

14. The device of claim 13, wherein the fluid flow regulator is formed of a polymer with non-thrombogenic properties.

15. The device of any one of claims 1 to 14 for use in extracting bodily fluid from a target site by:

    providing the device;
    penetrating the target site with the penetration member; and
    extracting bodily fluid from the target site.

16. The device of claim 15, which is adapted to extract ISF from the target site.

FIG. 1
(PRIOR ART)

FIG. 2
(PRIOR ART)

FIG. 3

410

400

402

404

5A

408

5A

FIG. 4

400

408  406  402  410

412  414

404

FIG. 5

EP 1 532 925 A1

FIG. 6

FIG. 7

11

FIG. 8

FIG. 9

1000

PROVIDING A DEVICE FOR EXTRACTING BODILY FLUID — 1010

PENETRATING A TARGET SITE WITH A PENETRATION MEMBER OF THE DEVICE — 1020

EXTRACTING BODILY FLUID FROM THE TARGET SITE VIA THE PENETRATION MEMBER AND A FLUID FLOW REGULATOR OF THE DEVICE — 1030

FIG. 10

**European Patent Office**

# EUROPEAN SEARCH REPORT

Application Number

EP 04 25 7169

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 266 625 A (LIFESCAN, INC) 18 December 2002 (2002-12-18) * paragraphs [0030], [0034] - [0036]; figure 1A * ----- | 1-7,12 | A61B5/15 |
| X | EP 1 360 933 A (LIFESCAN, INC) 12 November 2003 (2003-11-12) * paragraphs [0084], [0087], [0090] - [0095]; figure 6 * ----- | 1-7,12 | |
| A | US 6 569 128 B1 (CHRISTENSEN JAMES M ET AL) 27 May 2003 (2003-05-27) * column 3, lines 19-65 * * column 4, line 51 - column 5, line 6; figures 2A,4,5,7,8 * ----- | 1-14 | |

| | | | TECHNICAL FIELDS SEARCHED (Int.Cl.7) |
|---|---|---|---|
| | | | A61B A61M |

The present search report has been drawn up for all claims

| Place of search | Date of completion of the search | Examiner |
|---|---|---|
| Berlin | 10 February 2005 | Pohjamo, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 25 7169

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2005

| Patent document cited in search report | | Publication date | Patent family member(s) | | Publication date |
|---|---|---|---|---|---|
| EP 1266625 | A | 18-12-2002 | US | 2002188185 A1 | 12-12-2002 |
| | | | AU | 4445102 A | 19-12-2002 |
| | | | CA | 2390332 A1 | 12-12-2002 |
| | | | CN | 1391104 A | 15-01-2003 |
| | | | CZ | 20022024 A3 | 17-12-2003 |
| | | | EP | 1266625 A2 | 18-12-2002 |
| | | | JP | 2003038464 A | 12-02-2003 |
| | | | PL | 354419 A1 | 16-12-2002 |
| | | | US | 2003055326 A1 | 20-03-2003 |
| EP 1360933 | A | 12-11-2003 | US | 2003212344 A1 | 13-11-2003 |
| | | | CA | 2428349 A1 | 09-11-2003 |
| | | | CN | 1456888 A | 19-11-2003 |
| | | | EP | 1360933 A1 | 12-11-2003 |
| | | | JP | 2004000600 A | 08-01-2004 |
| US 6569128 | B1 | 27-05-2003 | US | 2002115966 A1 | 22-08-2002 |

For more details about this annex : see Official Journal of the European Patent Office, No. 12/82